# EUROPEAN PATENT SPECIFICATION

(11) **EP 2 274 326 B1**
(45) Date of publication and mention of the grant of the patent: **01.01.2014**
(21) Application number: 08873818.2
(22) Date of filing: 02.12.2008
(51) Int. Cl.: C07K 14/57, C12N 15/23, A61K 38/21, A61P 37/06

(54) **SUPPRESSOR OF THE ENDOGENOUS INTERFERON- GAMMA**
SUPPRESSOR VON ENDOGENEM INTERFERON-GAMMA
INHIBITEUR DE GAMMA INTERFÉRONS ENDOGÈNES

(30) Priority: 08.04.2008 BG 11010308
(43) Date of publication of application: 19.01.2011
(73) Proprietor: Tigo Gmbh, 65193 Wiesbaden (DE)
(72) Inventor: IVANOV, Ivan, 1517 Sofia (BG); NACHEVA, Genoveva, 1799 Sofia (BG); PETROV, Stefan, 1592 Sofia (BG); GRIGOLEIT, Hans- Guenther, D-65193 Wiesbaden (DE)
(74) Representative: Stefanova, Stanislava Hristova
(86) International application number: PCT/BG2008/000025
(87) International publication number: WO 2009/124362

(56) References cited:
- WO-A1-2006/099701
- WO-A2-2006/120580
- RU-C2- 2 268 749
- PETROV S ET AL: "Human interferon gamma: significance of lysine 88 for its biological activity", FEBS JOURNAL, vol. 273, no. Suppl. 1, June 2006 (2006-06), pages 92-93, XP9150853, & 31ST CONGRESS OF THE FEDERATION-OF-EUROPEAN-BIOCHEMICAL-SOCIETI ES (FEBS); ISTANBUL, TURKEY; JUNE 24 -29, 2006
- NACHEVA G ET AL: "Human interferon gamma: Significance of the C-terminal flexible domain for its biological activity", ARCHIVES OF BIOCHEMISTRY AND BIOPHYSICS, ACADEMIC PRESS, US, vol. 413, no. 1, 1 May 2003 (2003-05-01), pages 91-98, XP002373770, ISSN: 0003-9861, DOI: DOI:10.1016/S0003-9861(03)00113-9
- IVANOVA E ET AL: "Human interferon-gamma derivates to be used as potential drugs for treatment of multiple sclerosis", FEBS JOURNAL, vol. 275, no. Suppl. 1, June 2008 (2008-06), page 452, XP9150854, & JOINT CONFERENCE OF THE 33RD FEBS CONGRESS/11TH IUBMB CONFERENCE; ATHENS, GREECE; JUNE 28 -JULY 03, 2008

### SEQUENCE LISTING

<110> TIGO Gmbh
<120> SUPPRESSOR OF THE ENDOGENOUS HUMAN INTERFERON GAMMA
<130> 39BG08
<140> EP 08873818.2
   < 141 > 2008-12-02
< 160> 2
<170> Patentin version 3.3
<210> 1
   <211> 19
   <212> DNA
   <213> Artificial
<220>
   <223> forward primer
<400> 1
   cccaagctta tgcaggacc 19
<210> 2
   <211> 40
   <212> DNA
   <213> Artificial
<220>
   <223> Reverse primer
<220>
   <221 > misc_feature
   <222> (20)..(28)
   <223> n is a, c, g, or t
<400> 2
   gcttttcgaa gtcatcacgn nnnnnnnngt tgctattgaa 40

## Description

### Field of Invention

The present invention relates to suppressors of the endogenous human interferon-gamma (hIFN-γ) applicable for treatment of autoimmune diseases and for prevention graft arteriosclerosis and rejection of organs in allograft transplanted patients.

### Background of Invention

Immune system protects organism from pathogenic microorganism and foreign macromolecular substances. It identifies exogenous (foreign) bodies of molecular mass exceeding 5000 Da and produces specific antibodies for their neutralization. Immune response is regulated by numerous protein factors (cytokines) produced by specialized cells. In case of dysfunction (due to genetic disorders or inflection diseases) the immune system misidentifies certain body proteins as exogenous products and produces specific antibodies for their neutralization. This process lies in the etiology of a great number of autoimmune diseases such as asthma, rheumatoid arthritis, infertility, alopecia areata, multiple sclerosis (MS) and other neurodegenerative pathologies leading to disability and early death of about 2% of the human population. There is substantial evidence that immune responses resulting in IFN-γ production are associated also with the development of graft arteriosclerosis (GA) in allograft transplanted patients. The chronic rejection of allografts (including heart) is preceded by a luminal stenosis of the blood vessels and is denoted as "graft arteriosclerosis". As many as 50% of heart transplant recipients develop angiographically detectable GA three to five years following transplantation. The only treatment currently available for GA is retransplantation, which is costly and not always possible because of shortage of suitable donors. In that sense, the demand of therapeutics for treatment autoimmune diseases and GA is a major priority of the experimental medicine and pharmacy:

Inflammation reaction accompanying the autoimmune process is related with a lavish infiltration of the target tissue with T-lymphocytes and macrophages. They are represented by CD4⁺ cells producing Th1 proinflammatory cytokines such as interleukin 12 (IL-12) and hIFN-γ. The latter activates mononuclear cells to produce destructive substances like lymphotoxins and tumor necrosis factor alpha (TNF-α). It is shown that the pathogenesis of most autoimmune diseases is related with an abnormal production of hIFN-γ [1-6].

The overproduction of hIFN-γ (as in the case of MS) is inhibited by parenteral application of hIFN-β (see patents US082138, WO9530435, CA2361081). In other patents (RU2073522, RU2187332, RU02166959) mixtures of the three different interferons IFN-α, IFN-β and IFN-γ are recommended. It is reported that high dosage (8,000,000 IU/day) of IFN-β provoke unfavorable effects such as: a) T-cells proliferation blockade; b) neutralization of IL-12 thus enhancing the IFN-γ effect; c) decreased CD4+ (Th1, Th2) and CD8+ (Tcl) cell content without changing the Th1/Th2 cell ratio [7; d) decreased levels of both pro- and anti- inflammatory cytokines [8], etc.

Another approach for neutralization of the overproduced hIFN-γ in autoimmune disease is based on the application of humanized anti-IFN-γ antibodies (patent application WO0145747 and [9-11]). The anti-hIFN-γ antibodies, however, deprive the organism from hIFN-γ and their long-term application worsens the patients conditions.

An alternative way for decreasing the abnormal production of hIFN-γ in autoimmune diseases is based on the application of the so called "consensus interferons' IFN-con₁, IFN-con₂ and IFN-con₃, derivatives of the Type I hIFN-α, hIFN-β and hIFN-t (US0086534 and CA2299361). They show various side effects Including toxicity.

Proteins with aminoacid sequence partly coinciding with that of the human IFN-γ have been applied as antiviral, antiproliferative and immunomodulating agents (US4832959, WO02081507. AT393690). Their effects, however, cannot be presently assessed since the cited patents are not supported by clinical data.

It is known from the investigations of Petrov S. et al. [14] that hIFNc gene where a Gln codon is substituted for the Lys88 codon stabilizes the hIFNc/ receptor complex.

In a recent patent application, published as WO20061099701, it is described a new approach for inhibition of the endogenous hIFN-γ using inactive recombinant analogues of the hIFN-γ with preserved affinity to the hIFN-γ receptor. Subject of the patent application are three different inactive variants of hIFN-γ (a truncated hIFN-γ lacking 27 C-terminal aminoacids, a fusion hIFN-γ-hIFN-α1 protein and a UV inactivated hIFN-γ) which compete with the natural (endogenous) hIFN-γ for the hIFN-γ receptor. Thus, competing with the hIFN-γ receptor, the inactive variants of IFN-γ suppress its activity. Since that effect is dose dependant, the effect of endogenous IFN-y could be modulated by varying blood concentration of the hIFN-γ derivative proteins. This approach is applicable in the cases when the overproduction of endogenous hIFN-γ causes health problems as in the case of autoimmune diseases, including MS. Although these proteins are good competitors of hIFN-γ for its receptor, their tertiary structure is quite different in comparison with the native wild-type hIFN-γ, which in turn is a potential risk of formation of conformational antibodies. Related with this there is a need of new inactive variants of the hIFN-γ containing negligible changes in domains responsible for triggering the signal transduction pathway.

### Description

The invention relates to a suppressor of the endogenous human interferon-gamma applicable for treatment of autoimmune diseases and for prevention graft arteriosclerosis and rejection of organs in allograft transplanted patients. More precisely, the suppressor relates to inactive analogues of the hIFN-γ containing negligible changes in domains responsible for triggering the signal transduction pathway. The suppressor has preserved affinity to the hIFN-γ receptor but is incapable of activating the signal transduction pathway.

According to the present invention, the inactive analogues of the hIFN-γ are obtained by site directed mutagenesis of the hIFN-γ gene to substitute amino acids at positions 86, 87 and 88, which are known to play a key role in the hIFN-γ mediated signal transduction, with amino acids Glu, Met and Pro or Thr, Asn and Gly, respectively. Taking into consideration that the receptor binding sites are located at the N-terminal part of the molecule, this part remains unchanged.

With the aim of constructing hIFN-γ derivatives with amino acid substitutions at positions 86, 87 and 88, two PCR primers are synthesized. The forward primer (SEQ ID NO: 1) carries a *Hin*dIII site followed by a sequence identical with the 5' end of the hIFN-γ gene and the reverse primer bears a randomized region of 9 nucleotides (A, G, C or T) followed by an *Asu*II restriction site. Both restriction sites (*Hind*III and *Asu*II) are designed for cloning of the PCR products into the expression vector pJP₁R₃-hIFN-γ as described in W020061099701. The PCR reaction is carried out with a synthetic hIFN-γ gene coding for 143 aa protein (EP 0446582) as a template. Thus a combinatorial library is constructed containing a randomized region between nucleotides 218 and 227 in the hIFN-γ gene, corresponding to the aminoacid area 86-88.

The PCR fragments are purified by agarose gel electrophoresis, digested with both *Hin*dIII 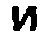 *Asu*II and cloned into the expression vector pJP₁R₃-hIFN-γ. A set of 162 clones is selected, plasmid DNA is isolated from each clone and the exact nucleotide sequence of the randomized region is determined by DNA sequencing. Thus 101 individual clones are chosen for testing of both antiviral and antiproliferative activities as well as for measuring their ability to compete with the wild-type hIFN-γ for the hIFN-γ receptor.

Two types of biological activity were measured: a) Antiviral activity (measuring the protective effect against the cytopathic action of the vesicular stomatitis virus (VSV) on the amniotic cell line WISH [20]) and b) Antiproliferative activity (as determined by the kynurenine bioassay [21]). The results presented in Table 1 show that both activities vary between 7,2x10⁵ and 3x10⁴ IU/mg for the constructs 19, 46-1. This is much lower in comparison with the activity of intact hIFN-γ (10⁷-10⁸ IU/mg). No biological activity was registered for the constructs 27, 36, 134,135 and 144.

**Table 1: Mutant HIFN-γ gene variants**

| Clone signature | Nucleotide sequence between nucleotides 218 and 227 | Aminoacids at positions 86, 87 and 88 | Specific activity of the mutant hIFN-γ proteins (IU/mg) | Competition with the wild type hIFN-γ |
|---|---|---|---|---|
| 19 | TCC ACT TTT | Ser Thr Phe | 7.2×10⁵ | + |
| | | | **2×10⁴ | |
| 27 | GAA ATG CCC | Glu Met Pro | 0 | +++ |
| 36 | CTG TGT CCC | Leu Cys Pro | 0 | ++ |
| 46-1 | ACC CTC CTC | Thr Leu Leu | *3,0×10⁴ | + |
| 134 | ACC AAT GGT | Thr Asn Gly | 0 | +++ |
| 136 | GTT TCC CCC | Val Ser Pro | 0 | + |
| 144 | TGC GCC CCT | Cys Ala Pro | 0 | + |

| | | | | |
|---|---|---|---|---|
| *antiproliferative activity; **antiviral activity; "+" competition with the wild type hIFN-γ: "+++" - high competition, "++" - intermediate competition, "+" - low competition | | | | |

All constructs without or with decreased biological activity are further assayed for their ability to compete with intact (wild-type) hIFN-γ for its receptor using WISH cells as a test system. To this end the mutant proteins are mixed in equimolar amounts in sterile bacterial lysates with purified hIFN-γ and the antiproliferative activity of the mixtures is measured by the kynurenine bioassay using wild-type hIFN-γ as a standard. The results must be interpreted as follows: In case that the mutant protein has the same affinity to the hIFN-γ receptor as that of the wild type hIFN-γ and zero antiproliferative activity, the activity of the equimolar mixture of both substances should be 50% of that of the control (pure wild type hIFN-γ). As it is shown in Table 1, the strongest competitive effect is expressed by the constructs showing zero antiproliferative activity (constructs 27 and 134).

The advantages of the hIFN-γ suppressor according to the invention cover several aspects: a) The hIFN-γ mutants represent negligibly minor modified (3 aminoacid substitutions) human proteins. They resemble allelic variants of the hIFN-γ and therefore they should not be immunogenic; b) The inactive hIFN-γ derivatives occupy the hIFN-γ receptor via a reversible manner and can be replaced by higher concentrations of intact hIFN-γ; c) The equal opportunity of both molecules (mutant and wild-type hIFN-γ) to bind the hIFN-γ receptor makes it possible to control the extent of inhibition of the activity of the endogenous hIFN-γ by varying the concentration of the suppressors in the bloodstream; d) Unlike other approaches for suppressing hIFN-γ activity (inhibition of its biosynthesis or irreversible neutralization, e.g. by specific monoclonal antibodies) the inactive derivatives of the hIFN-γ do not deprive the organism of the vitally important endogenous hIFN-γ.

The suppressor, according to the present invention, is designed for manufacturing of medicaments for inhibition the activity of the endogenous hIFN-γ, produced in high concentrations in patients with autoimmune diseases such as *Multiple sclerosis, Alopecia areata, Myastenia gravis* as well as for graft arteriosclerosis in post-transplanted patients.

The following examples illustrate the present invention without limiting its scope and spirit :

### Example 1: Construction of hIFN-γ derivative proteins with aminoacid substitutions at positions 86, 87 and 88.

Recombinant proteins derivative of the hIFN-γ with aminoacid substitutions at positions 86, 87 and 88 are prepared by PCR mutagenesis of a synthetic hIFN-γ gene using appropriate primers. The latter are synthesized on a Cyclon Plus (milliGene) synthesizer by the phosphorimidite methods and purified on a 15% polyacrylamide gel [22]. Two primers (forward and reverse) are synthesized and their primary structure is presented on the sequence listing. The forward primer (SEQ ID- NO: 1) is designed to introduce a *Hin*dIII site and the reverse primer contains a randomized 9 nucleotide long region plus an *Asu*II site (SEQ ID NO: 2). The two (*Hin*dIII and *Asu*II) restriction sites are to be used for cloning into the expression vector pJP₁R₃-hIFN-γ. The hIFN-γ is mutagenized under PCR conditions presented in Tables 2 and 4.

**Table 2: PCR conditions for primers SEQ ID NO:1 and SEQ ID NO:2**

| **Programme** | **Number of cycles** | **Time (min)** | **Temperature (°C)** |
|---|---|---|---|
| I | 1 | 5 | 94 |
| II | 5 | 0.5 | 94 |
| | | 0.5 | 38 |
| | | 0.5 | 74 |
| III | 35 | 0.5 | 94 |
| | | 0.5 | 55 |
| | | 0.5 | 74 |
| IV | 1 | 10 | 74 |

The PCR fragments are purified by electrophoresis in 1.5% Agarose Type II (Sigma) gel, hydrolyzed by *Hin*dIII and *Asu*II and cloned into the expression vector pJP₁R₃-hIFN-γ pre-digested with the same enzymes (see WO20061099701). To this end 20 µg plasmid (vector) DNA is dissolved in 150 µl *Hin*dIII buffer and digested with 20 U *Hin*dIII for 3 h at 37°C. Reaction mixture is treated consecutively with phenol and chloroform and DNA is precipitated with 3 v/v of ethanol at -20 °C. The precipitate is dissolved in 150 µl Asull buffer and digested with 20 U *Asu*II for 3 h at 37°C. The linear vector is dephosphorylated by calf intestinal alkaline phosphatase (CIAP, Boehringer Mannhein), purified by agarose gel electrophoresis and mixed in T4 DNA ligase buffer with the PCR fragments at a ratio 3:1. The ligase reaction is carried out overnight at 4 °C and used for transformation of competent *E.coli* LE392 cells.

The transformed cells are grown in standard Luria-Bertani (LB) broth and/or LB-agar containing 50 µg/ml ampicillin and 10 µg/ml tetracycline. A set of 162 clones were selected, plasmid DNA was isolated from each clone and the exact nucleotide sequence of the randomized region was determined by DNA sequence analysis. Thus the number of individual clones was reduced to 101 and they were all tested for production of hIFN-γ derivative proteins. The level of expression of the latter was determined by ELISA using hIFN-γ specific monoclonal antibodies.

The hIFN-γ derivative proteins are purified in two steps by Octyl-Sepharose and CM-Sepharose (Pharmacia) chromatography as already described (EP0446582). Two biological activities, antiviral and antiproliferative, were determined for the hIFN-γ derivative proteins. The antiviral activity (expressed in International Units) is measured by the protective effect of hIFN-γ against the cytopathic action of the vesicular stomatitis virus (VSV) on the amniotic cell line WISH [12] and the antiproliferative activity was determined by the kynurenine bioassay [13].

The data presented in Table 1 show that most of the mutant hIFN-γ proteins have either decreased or completely lost antiviral and antiproliferative activities.

### Example 2: Examination of the suppressor activity of mutant hIFN-γ proteins

Capability of mutant hIFN-γ derivative proteins of competing with the wild-type hIFN-γ for the hIFN-γ receptor is examined on the amniotic cell line WISH (enriched in hIFN-γ receptors). The test is based on measuring the decrease in antiproliferative activity of standard (wild-type) hIFN-γ in the presence of mutant hIFN-γ derivative proteins. The antiproliferative activity itself is determined by the kynurenine bioassay [13] based on the hIFN-γ induction of Indoleamine-2,3- dioxygenase (IDO), which is the first and rate-limiting enzyme in the tryptophan catabolism. IDO catalyzes oxidative cleavage of tryptophan to N-formylkynurenine. Following a hydrolysis step, the latter is transformed into kynurenine which gives with the Ehrlich's reagent a yellow-colored compound absorbing at 490 nm. It is known that the amount of produced kynurenine is directly proportional to the concentration of hIFN-γ used for cell activation.

To measure the suppressor activity, mutant proteins are mixed in equimolar amounts in sterile bacterial lysates with purified hIFN-γ and the antiproliferative activity of the obtained mixtures is determined by the kynurenine bioassay using wild-type hIFN-γ as a standard. Experimentally, clear cell lysates of *E. coli* LE392 cells transformed with plasmids expressing mutant hIFN-γ proteins are prepared after cultivation in LB broth supplemented with 50 µg/ml ampicillin to a cell density of A₅₉₀=0.7. Samples of 2 OD₅₉₀ cells are centrifuged, the cells are resuspended in 1 ml 0.14 M NaCl, 10 mM Tris pH 8.0, 0.1 mM PMSF and disrupted by sonication. The lysates are cleared by centrifugation at 12000 rpm for 15 min at 4 °C and used for further analyses.

Total protein content is determined by Bradford using bovine serum albumin (fraction V) as a standard and the samples are diluted by PBS (14.7 mM Na₂CO₃ 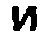 34 mM NaHCO₃, pH 9.6) to a final concentration of 27 µg/ml protein. Samples of 50 µl are added (11 times per sample) to PVC 96 well microplates (Costar Ltd., USA), incubated overnight at 4°C and the content of hIFN-γ or hIFN-γ derivative proteins is measured by ELISA using hIFN-γ specific monoclonal antibodies.

To measure the suppressive effect of the hIFN-γ mutant proteins against binding of wild-type hIFN-γ to the cell receptors, clear cell lysates are serially diluted and samples of 50 µl are mixed with 50 µl of standard hIFN-γ and added to PVC 96 well microplates. WISH cell suspension (50 µl) in MEM Eagle medium supplemented with HEPES, 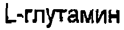 and 2% BFS is added, mixed with 50 µl L-tryptophan and the kynurenine bioassay is performed as already described [13]. The final concentration of the standard hIFN-γ in the analyzed samples is 25 IU/ml, 50 IU/ml and 100 IU/ml, corresponding to 0.027 nmol, 0.055nmol and 0.11 nmol respectively. Samples containing standard hIFN-γ (only) were used as Positive control and clear cell lysates obtained from host (non-transformed *E*. *coli* LE392) cells were used as negative controls in this assay.

The data presented in Table 1 show that the constructs with zero antiproliferative activity (constructs 27 and 134) demonstrate strongest suppressive effect.

### REFERENCES

1. Johnson, K.P. (1988) Treatment of multiple sclerosis with various Interferons. The Cons. Neurology, 38 (suppl. 2) 52-64.
2. Martino, G., Moiola, L., Brambilla, E., Clementi. E., Comi, G., Grimaldi. L.M. (1995). Interferon gamma induces T lymphocyte proliferation in multiple sclerosis via a Ca2+-dependent mechanism. J. Neuroimmunol. 62, 169-176.
3. Vartanian, V., Li. Y., Zhao. M., Stefansson, K. (1995) Interferon-gamma-induced oligodendrocyte cell death: implications for the pathogenesis of multiple sclerosis. Mol. Med. 1, 732-743.
4. Tellides. G., Pober, J. (2007) Interferon-γ axis in graft arteriosderosis. Circ. Res. 100, 622-632.
5. Panitch, H.L., Hirsch, RL., Schindler. J., Johnson, K.P. (1987) Treatment of multiple sclerosis with gamma interferon: Exacerbations associated with activation of the immune system. Neurology 37, 1097-1102.
6. Beck, J., Rondot, P., Catinot, L., Falcoff, E., Kirchner, H., Wietzerbin, J. (1988) increased production of interferon gamma and tumor necrosis factor precedes clinical manifestation in multiple sclerosis; do cytokines trigger off exacerbations? Acta Neurol. Scand. 78, 318-323.
7. Furlan, R, Bergamini A., Lang. R, Brambilla, E., Franciotta, D., Martinelli, V., Comi, G., Paninam P., Martino. G. (2000) interferon-beta treatment in multiple sclerosis patients decreases the number of circulating T cells producing interferon-gamma and interleukin-4.J. Neuroimmunol. 111, 86-92.
8. Khademi, M., Wallstrom, E., Andersson, M., Piehl, F., Di Marco. R., Olsson,T. (2000) Reduction of both pro- and anti-inflammatory cytokines after 6 months of interferon beta-1a treatment of multiple sclerosis. J. Neuroimmunol. 103, 202-210.
9. Skurkovich, S., Boiko, A, Bellaeva, L, Buglak. A.. Alekseeva, T., Smimova, N., Kulakova, O., Tchechonin, V., Gurova, O., Deomina, T., Favorova, O.O., Skurkovic, B., Gusev, E. (2001) Randomized study of antibodies to IFN-gamma and TNF-alpha in secondary progressive multiple sclerosis. Mutt. Scler. 7, 277-284:
10. Skurkovich, B.. Skurkovich, S. (2003) Anti-interferon-gamma antibodies in the treatment of autoimmune diseases. Curr. Opin. Mol. Ther. 5, 52-57.
11. Espejo, C., Penkowa, M., Saez-Torres, I., Xaus, J., Celada, A., Montalban, X., Martinez-Caceres, EM. (2001) Treatment with anti-interferon-gamma monoclonal antibodies modifies experimental autoimmune encephalomyelitis in interferon-gamma receptor knockout mice. Exp. Neurol. 172, 460-468
12. Forti, R. L., Schuffman. S. S., Davies. H. A. and Mitchell. W. M. (1986) Objective antiviral assay of the interferons by computer assisted data collection and analysis, Methods in Enzymol. 119, 533-540.
13. Boyanova, M., Tsanev, R. and Ivanov, 1. (2002) A modified kynurenine bloassay for quantitative determination of human Interferon gamma. Analyt. Biochem. 308, 178-181.
14. Petrov S et al: "Human interferon gamma: significance of lysine 88 for its biological activity". FEBS Journal, vol. 273, no. Suppl. 1, June 2006, p. 92-93, XP9150853, &31-st Congress of The Federation- of- European-Biochemical- Societies (FEBS); Istanbul, Turkey. June 24- 29, 2006

## Claims

1. Suppressor of endogenous human interferon-gamma (hIFN-γ) based on inactive analogues of the hIFN-γ with preserved affinity to the hIFN-γ receptor, characterized with that it represents derivatives of the hIFN-γ where amino acids at positions 86, 87 and 88 are substituted with Glu, Met and Pro or Thr, Asn and Gly, respectively.

2. Suppressor of endogenous hIFN-γ according to claim 1, characterized with that the amino acid substitutions are introduced in the hIFN-γ gene by a forward primer SEQ ID No:1 and a reverse primer SEQ ID No:2.

3. Use of the suppressor of endogenous hIFN-γ according to claims 1 and 2 for manufacturing of a medicament for treatment of diseases associated with impaired activity of endogenous hIFN-γ.

4. Use of the suppressor of endogenous hIFN-γ according to claim 3 for manufacturing of a medicament for treatment of autoimmune diseases such as *Multiple sclerosis, Alopecia areata and Myastenia gravis.*

5. Use of the suppressor of endogenous hIFN-γ according to claim 3 for manufacture of a medicament for prevention of graft arteriosclerosis and rejection of organs in allograft transplanted patients.

## Patentansprüche

1. Hemmstoff des endogenen humanen Interferon-gamma (hIFN-γ) auf der Basis inaktiver Analoge des hIFN-γ mit erhaltener Affinität zum hIFN-γ Rezeptor, dadurch charakterisiert, dass dieser Derivate des hIFN-γ darstellt, in welchen die Aminosäuren in den Positionen 86,87 und 88 jeweils durch Glu, Met, und Pro oder Thr, Asn und Gly substituiert werden.

2. Hemmstoff des endogenen humanen Interferon-gamma (hIFN-γ) entsprechend Anspruch 1, dadurch charakterisiert, dass die Aminosäuresubstitionen in das hIFN-γ Gen durch eine Forward Primer SEQ ID No:1 und eine Reverse Primer SEQ ID No:2 eingeführt werden.

3. Verwendung des Hemmstoffes des endogenen hIFN-γ entsprechend Ansprüchen 1 und 2 zwecks Herstellung eines Medikamentes zur Behandlung von Krankheiten, welche assoziiert sind mit einer beeinträchtigenden Aktivität des endogenen hIFN-γ.

4. Verwendung des Hemmstoffes des endogenen hIFN-γ entsprechend Anspruch 3 zwecks Herstellung eines Medikamentes zur Behandlung von Autoimmunerkrankungen wie Multiple Sklerose, Alopecia areata und Myasthenia gravis.

5. Verwendung des Hemmstoffes des endogenen hIFN-γ entsprechend Anspruch 3 zwecks Herstellung eines Medikamentes zur Prävention der Transplantationsvaskulopathie und Organabstoßung bei Patienten mit Allograften.

## Revendications

1. Antagoniste du récepteur aux gamma interférons endogènes humains (hIFN-γ) basé sur des analogues inactifs d'hIFN-γ ayant une affinité conservée pour le récepteur d'hIFN-γ, **caractérisé en ce qu'**il est représenté par des dérivés d'hIFN-γ dans lesquels les acides aminés dans les positions 86, 87 et 88 ont été respectivement substitués par Glu, Met et Pro, ou par Thr, Asn et Gly.

2. Antagoniste du récepteur aux hIFN-γ endogènes selon la revendication 1, **caractérisé en ce que** les substitutions d'acides aminés sont introduites par l'amorce sens SEQ ID N° : 1 et par l'amorce anti-sens SEQ ID N° : 2, dans le gène de l'hIFN-γ.

3. Utilisation de l'antagoniste du récepteur aux hIFN-γ endogènes selon les revendications 1 et 2 pour la fabrication d'un médicament destiné au traitement de maladies associées à des troubles de l'activité des hIFN-γ endogènes.

4. Utilisation de l'antagoniste du récepteur aux hIFN-γ endogènes selon la revendication 3 pour la fabrication d'un médicament destiné au traitement de maladies auto-immunes telles que la sclérose en plaques, l'alopécie areata et la myasthénie auto-immune.

5. Utilisation de l'antagoniste du récepteur aux hIFN-γ endogènes selon la revendication 3 pour la fabrication d'un médicament destiné à la prévention de l'artériosclérose due à une greffe et du rejet d'organes chez des patients transplantés d'une allogreffe.
